# EUROPEAN PATENT SPECIFICATION

(11) **EP 2 078 504 B1**
(45) Date of publication and mention of the grant of the patent: **21.12.2016**
(21) Application number: 09150213.8
(22) Date of filing: 08.01.2009
(51) Int. Cl.: A61B 17/3203, F04B 43/04

(54) **Fluid ejecting apparatus, surgical operation instrument**
Flüssigkeitsausgabevorrichtung, chirurgisches Operationsinstrument
Appareil d'éjection de fluide, instrument chirurgical

(30) Priority: 11.01.2008 JP 2008004046; 09.10.2008 JP 2008262494
(43) Date of publication of application: 15.07.2009
(73) Proprietor: Seiko Epson Corporation, Shinjuku-ku Tokyo (JP)
(72) Inventor: Seto, Takeshi, Nagano 392-8502 (JP); Kawasumi, Kazuo, Nagano 392-8502 (JP)
(74) Representative: Hoffmann Eitle

(56) References cited:
- WO-A1-00/44292
- JP-A- 2005 152 127
- JP-A- 2006 342 723
- US-A- 5 171 132
- US-A1- 2002 162 595
- US-A1- 2005 074 340
- US-A1- 2005 175 490
- US-A1- 2006 013 710

## Description

### BACKGROUND

### 1. Technical Field

The present invention relates to a fluid ejecting apparatus, having a check valve for opening and closing an inlet channel which is in communication with a fluid chamber and a surgical operation instrument having the fluid ejecting apparatus.

### 2. Related Art

A surgical operation with fluid ejected in pulses enables incision of an organ substance while conserving a vascular structure such as blood vessels. In addition, an associated damage given to anatomy other than the portion to be incised is light, and hence a burden to a patient is lightened. Also, since the amount of bleeding is small, the blood does not block the visibility, and hence a quick surgical operation is enabled. Therefore, it is clinically applied in many cases to hepatic resection or the like which is associated with a difficulty due to bleeding specifically from small blood vessels.

As an example of the fluid ejecting apparatus for incising or excising anatomy, there is a fluid ejecting apparatus which ejects fluid from a fluid ejecting opening in pulses by reducing the capacity of the fluid chamber (pump chamber body) by a diaphragm. In the fluid ejecting apparatus in this configuration, there is a known type of the fluid ejecting apparatus in which the check valve is provided between the fluid chamber and the inlet channel for supplying the fluid to the fluid chamber, and the check valve is opened when supplying the fluid and is closed when reducing the capacity of the fluid chamber (see JP-A-2005-152127 (p. 7, Fig. 1)).

In the configuration disclosed in JP-A-2005-152127, the check valve is directly provided at an inlet connecting tube having the inlet channel provided therein. A communicating portion between the inlet connecting tube and the fluid chamber is set to be extremely small in order to increase the capacity reduction ratio, and hence the check valve by itself is extremely small, so that manufacturing or handling of the check valve is difficult.

In addition, document US 5,171,132 A discloses a fluid ejecting apparatus as defined in the preamble of appended claim 1. Reference may also be made to the pump with diaphragm and check valve, which is disclosed in document JP 2006-342723 A.

### SUMMARY

An advantage of some aspects of the invention is achieved by the following modes or embodiments.

A fluid ejecting apparatus according to the invention is as defined in appended claim 1.

In this configuration, since the check valve is integrally formed when manufacturing at least one of the first and second diaphragms, it is not necessary to manufacture the minute check valve as a single unit, so that the manufacture is simplified. Since the check valve is attached by attaching said at least one of the first and second diaphragms to the fluid chamber, advantages such as easy-to-handle and improved assembleability are achieved.

By forming said at least one of the first and second diaphragms and the check valve integrally, the relative positional relationship between said at least one of the first and second diaphragms and the check valve is enhanced, thereby achieving an advantage such that the positional accuracy between the opening and closing inlet channel and the check valve is improved.

With the provision of the check valve, the fluid is prevented from flowing reversely to the inlet channel when the fluid chamber is reduced by at least the first diaphragm, and the internal pressure in the fluid chamber is increased to a required level, so that the fluid ejecting efficiency is improved.

Preferably, the check valve is formed with a slit.

In this configuration, formation of the check valve is achieved by providing a substantially U-shaped slit on said at least one of the first and second diaphragms. Since said at least one of the first and second diaphragms is formed of a thin plate, said at least one of the first and second diaphragms and the check valve can be formed with the same machining process such as pressing or etching.

Preferably, the check valve includes a thinned portion at a bent portion in the direction of thickness thereof.

In this configuration, with the provision of the thinned portion at the bent portion of the check valve, the check valve is bent with high-sensitivity in response to the pressure difference between the fluid chamber and the inlet channel while having a thickness suitable for said at least one of the first and second diaphragms, closes the inlet channel when reducing the capacity of the fluid chamber, and opens the inlet channel when the capacity of the fluid chamber is restored to an initial state.

Preferably, the check valve is fixed at a position apart from a range where the thinned portion is formed.

In this configuration, since the check valve is bent at the thinned portion, displacement due to bending is not transmitted to the fixing portion. Therefore, since an infinitely small gap is not formed between the fixing portion and a fixed portion when the check valve is bent, such events that fine dust is clogged in the infinitely small gap due to repetition of the bending operation and hence the closing property of the check valve is deteriorated, or a stress is intensively applied to a fulcrum due to the presence of the dust and hence the check valve is broken are avoided, so that the durability is improved.

Preferably, a communicating portion having a size in a range which does not hinder the operation of the check valve is provided at an end of the fluid chamber on the side of the inlet channel.

In this configuration, with the provision of the communicating portion continuing to the fluid chamber, the operating range of the check valve is secured in a small range without giving a significant influence on the capacity of the fluid chamber, which is advantageous for downsizing.

Preferably, the inlet channel is disposed on the opposite side from the fluid chamber with respect to said at least one of the first and second diaphragms so as to apart therefrom, and the check valve is disposed in a communication channel which communicates the fluid chamber and the inlet channel.

In this configuration, since the check valve can be formed in the same plane as said at least one of the first and second diaphragms, the shape is simplified, and the relative positional accuracy with respect to the opening and closing communication channel is improved, so that the inlet channel is reliably closed.

Preferably, the communication channel is provided at a position apart from a peripheral edge of the fluid chamber, and is in communication with the fluid chamber via a connecting hole.

In this configuration, a notch is not present on the peripheral edge of the fluid chamber and hence a fixing surface having an entirely continuing circumference is provided for said at least one of the first and second diaphragms, so that the entire circumference of said at least one of the first and second diaphragms is fixed, the amount of displacement of said at least one of the first and second diaphragms is stabilized, and the amount of reduction of the capacity of the fluid chamber is also stabilized.

Preferably, the check valve projects to a position covering from an end portion of said at least one of the first and second diaphragms to the communicating portion.

In this configuration, the shape of the check valve is simplified and an easy-to-manufacture structure is achieved.

Preferably, the check valve has a width at the bent portion smaller than portions other than the bent portion.

When the width of the bent portion is reduced, the rigidity of the bent portion is further reduced, so that the followability to open and close the inlet channel in response to the pressure difference between the fluid chamber and the inlet channel is improved.

Preferably, the check valve includes two supporting arms projecting from an end of said at least one of the first and second diaphragms in the direction toward the communication channel, and a closed portion positioned between the two supporting arms and projecting from distal ends of the two supporting arms toward the end of said at least one of the first and second diaphragms, and the sum of the widths of the supporting arms are smaller than the width of the closed portion, and the closed portion opens and closes the communication channel by the supporting arms being bent.

In this configuration, by setting the widths of the supporting arms than the width of the closed portion, the rigidity of the bent portion is reduced, and the followability to open and close the communication channel is improved.

Since the supporting arms are disposed on both sides of the closed portion, the good stress balance in bending operation is achieved, and hence inclination with respect to the communication channel due to torsion or the like is restrained, so that the communication channel is reliably closed.

Preferably, the inlet channel is provided substantially in parallel with a bottom surface of the fluid chamber, and is brought into communication directly with the fluid chamber, and the check valve is extended by being bent in the substantially vertical direction with respect to the fixing surface of said at least one of the first and second diaphragms, and opens and closes the inlet channel.

In this configuration, since the inlet channel is linearly communicated with the fluid chamber, the resistance of the fluid in the communicating portion between the inlet channel and the fluid chamber is reduced to allow the fluid to flow in the fluid chamber easily, and the reduction of the dimension in the direction of thickness is achieved.

In addition, as recited in appended claim 1, the fluid chamber is defined by a frame-shaped spacer having an opening penetrating in the direction of thickness, the first and second diaphragms are disposed on both surfaces of the spacer for sealing the opening, and the check valve is formed on at least one of the first and second diaphragms.

In this configuration, since the first and second diaphragms are provided on opposed surfaces of the one fluid chamber, the capacity reduction ratio of the fluid chamber is enhanced, and since the check valve is provided, the reverse flow of the fluid is prevented, and the pressure in the fluid chamber is enhanced.

Preferably, two inlet channels communicating with the fluid chamber are provided, and two check valves are provided on the first and second diaphragms and open and close the two inlet channels respectively.

In this configuration, with the provision of the two inlet channels for the one fluid chamber, the amount of supply of the fluid to the fluid chamber is increased. Accordingly, since the fluid supply pressure from the outside may be reduced, and hence the fluid supply is achieved in a simple method such as hanging a liquid bag as a fluid supply source of the fluid at a position higher than the fluid ejecting apparatus.

Preferably, the inlet channel is defined by a groove provided along the outer periphery of the fluid ejecting unit and an inner periphery of the fluid supply tube fitted so as to cover the groove.

In this configuration, since the inlet channel is defined by the groove provided on the outer periphery of the fluid ejecting unit, the inlet channel is formed by cutting process or the like, so that it can be formed easily in comparison with forming the same with a thin tube. There is also an advantage such that the flexibility in size of the cross-sectional area of the inlet channel is increased, so that adjustment in increase and decrease of the amount of liquid supply is easily achieved.

Preferably, at least one of the first and second diaphragms is provided with a waveform structure having projections and depressions in the direction of thickness of said at least one of the first and second diaphragms around a piezoelectric element for displacing said at least one of the first and second diaphragms.

With the provision of the waveform structure, the inplane stress generated when said at least one of the first and second diaphragms is displaced is restrained, so that the capacity reduction of the fluid chamber is efficiently enabled.

Preferably, a surgical operation instrument includes the fluid ejecting apparatus according to the invention.

In this configuration, a compact surgical operation instrument having a high fluid ejecting efficiency is achieved by the employment of the fluid ejecting apparatus having integrally formed diaphragm and check valve.

### BRIEF DESCRIPTION OF THE DRAWINGS

The invention will be described with reference to the accompanying drawings, wherein like numbers reference like elements.
Fig. 1 is an explanatory drawing showing an example of a schematic configuration of a fluid ejecting apparatus according to a first embodiment.
Fig. 2 is a side cross-sectional view of a fluid ejecting unit according to the first embodiment.
Fig. 3A is a plan view showing the fluid ejecting unit in a state of viewing through the upper frame according to the first embodiment.
Fig. 3B is a partial plan view showing the shape of a check valve in plan view in an enlarged scale.
Fig. 4 is a partial cross-sectional view showing an operation of the check valve according to the first embodiment.
Fig. 5 is a partial plan view showing the check valve according to another example of the first embodiment.
Fig. 6A is a perspective view showing a part of a lower frame of the fluid ejecting unit according to a second embodiment.
Fig. 6B is a side cross-sectional view showing a part of the fluid ejecting unit according to the second embodiment.
Fig. 7A is a partial plan view showing a diaphragm of the check valve according to Example 1 in the second embodiment.
Fig. 7B is a cross-sectional view showing a part of the fluid ejecting unit of the check valve according to Example 1 in the second embodiment.
Fig. 8 is a partial plan view showing the check valve according to Example 2 in the second embodiment.
Fig. 9 is a partial plan view showing the check valve according to Example 3 in the second embodiment.
Fig. 10 is a partial plan view showing the check valve according to Example 4 in the second embodiment.
Fig. 11 is a side cross-sectional view showing a part of the fluid ejecting unit according to a third embodiment.
Fig. 12 is a partial plan view showing the check valve according to Example 1 in the third embodiment.
Fig. 13 is a partial cross-sectional view showing a part of the fluid ejecting apparatus according to Example 1 in the third embodiment.
Fig. 14 is a partial plan view showing the check valve according to Example 2 in the third embodiment.
Fig. 15 is a side cross-sectional view showing the fluid ejecting unit according to a fourth embodiment.
Fig. 16A is a perspective view of a spacer according to the fourth embodiment.
Fig. 16B is a partial perspective view showing another example of the spacer according to the fourth embodiment.
Fig. 17A is a side cross-sectional view of the fluid ejecting unit according to a fifth embodiment.
Fig. 17B is a cross-sectional view taken along the line C-C in Fig. 17A.
Fig. 18 is a plan view showing a configuration of the diaphragm according to a sixth embodiment.
Fig. 19 is a cross-sectional view taken along the line A-A in Fig. 18.

### DESCRIPTION OF EXEMPLARY EMBODIMENTS

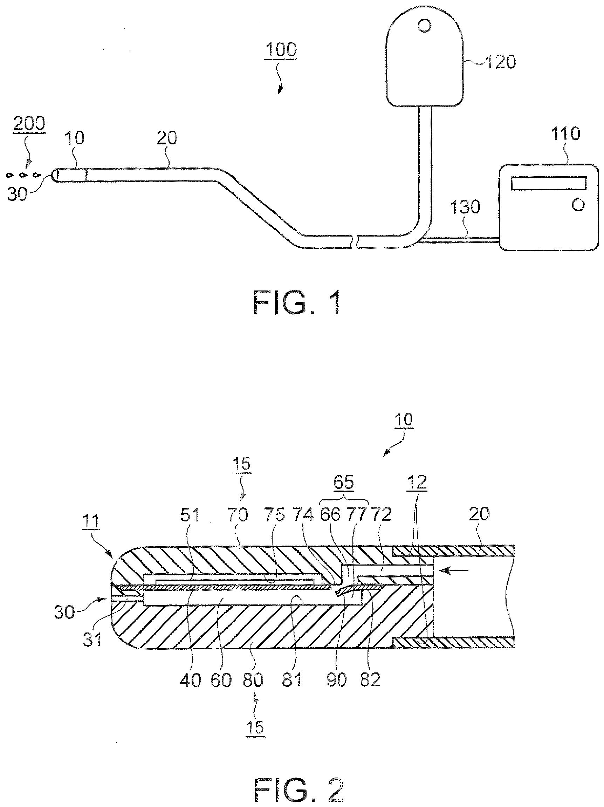

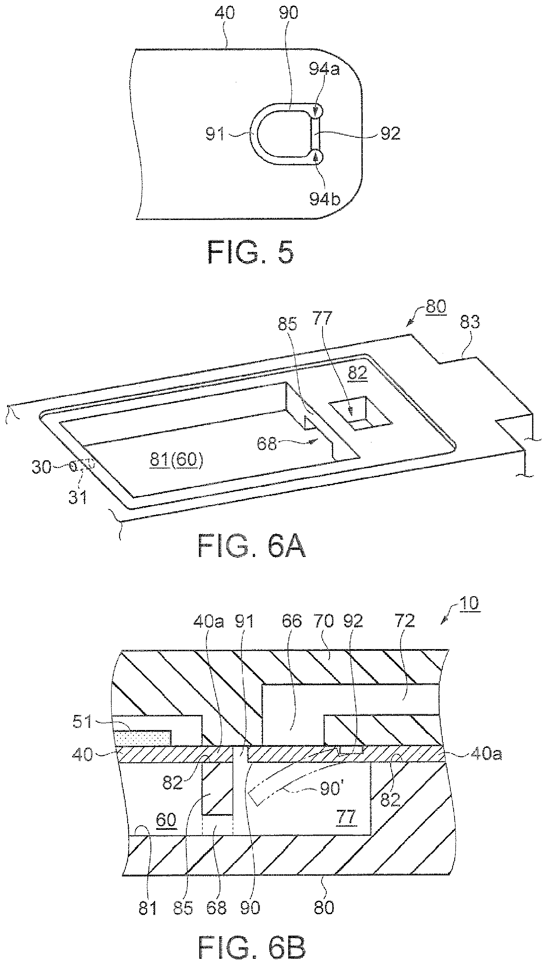

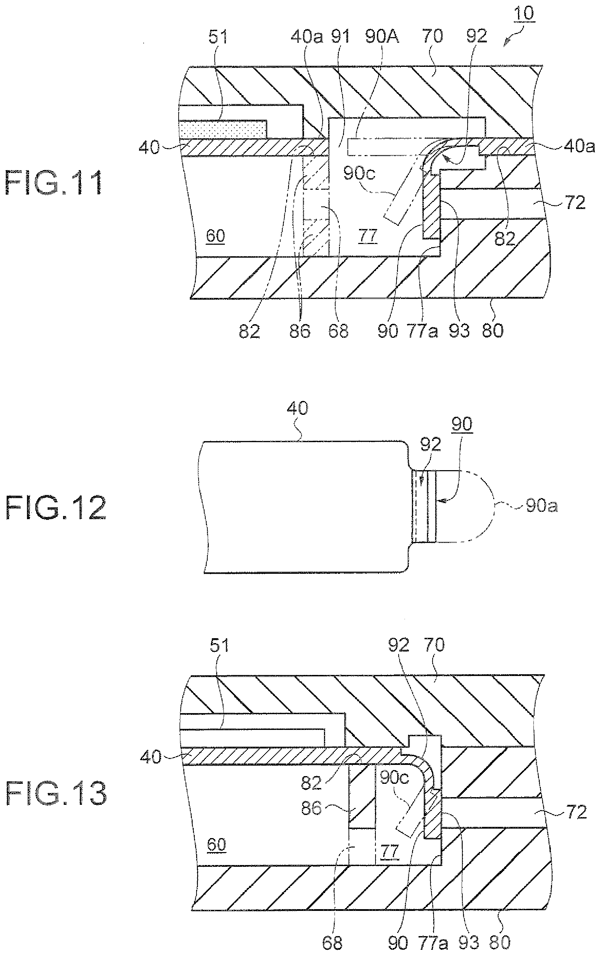

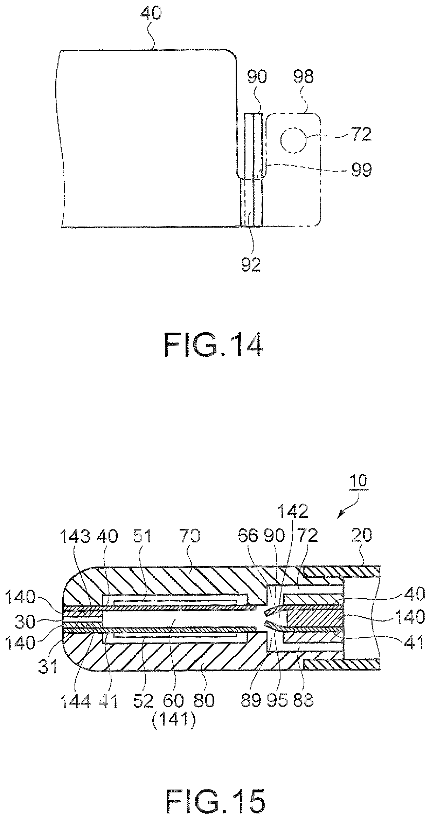

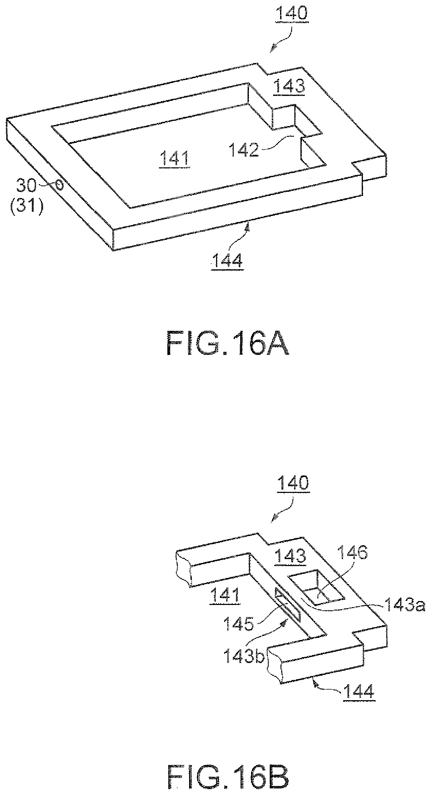

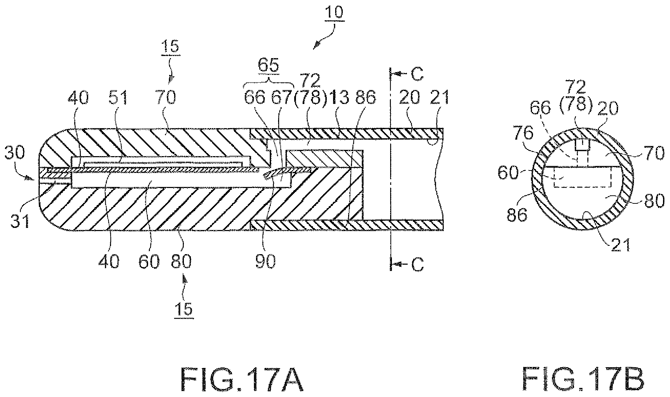

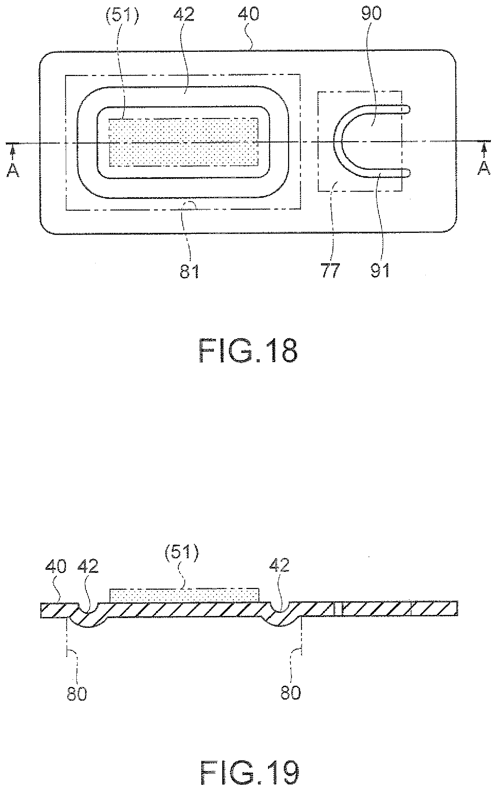

Referring now to the drawings, illustrative embodiments and embodiments of the invention will be described. Fig. 1 to Fig. 5 show a fluid ejecting apparatus according to a first embodiment out of the scope of the claims, Fig. 6 to Fig. 10 show a fluid ejecting apparatus in a second embodiment out of the scope of the claims, Fig. 11 to Fig. 14 show a fluid ejecting apparatus in a third embodiment out of the scope of the claims, Figs. 15 and 16 show a fourth embodiment falling within the scope of the claims, Fig. 17 shows a fifth embodiment falling within the scope of the claims when comprising two diaphragms, and Figs. 18 and 19 show a fluid ejecting apparatus according to a sixth embodiment falling within the scope of the claims when comprising two diaphragms. Drawings referred to in the description shown below are multiple drawings in which members and the lateral and vertical scale reductions are different from the actual conditions for the sake of convenience.

The fluid ejecting apparatus according to the illustrative embodiments and embodiments of the invention may be applied to various cases such as drawing using ink or the like, cleaning of a precise substance and structure, cutting or excision of a substance, and a surgical knife. However, in the embodiments shown below, a fluid ejecting apparatus which is suitable for installing a catheter at a distal end for inserting into a blood vessel for removing the thrombus or the like, or a fluid ejecting apparatus suitable for incising or excising anatomy are exemplified for description. Therefore, fluid used in the embodiments includes water, physiologic saline, chemical solution or the like and, these types of fluid are referred to as liquid hereinafter.

### First Embodiment

Fig. 1 is an explanatory drawing showing an example of a schematic configuration of a fluid ejecting apparatus according to a first embodiment. In Fig. 1, a liquid ejecting apparatus 100 basically includes an infusion solution bag 120 that accommodates liquid and serves as a liquid supply source, a drive control unit 110, a fluid ejecting unit 10 which converts the flow of the liquid into a pulsation flow, and a fluid supply tube 20 that communicates the infusion solution bag 120 and the fluid ejecting unit 10. The fluid ejecting unit 10 changes the flow of the liquid into pulsation, and ejects as liquid drops 200 from a fluid ejection opening 30 quickly in pulses. The fluid supply tube 20 may be a catheter.

The drive control unit 110 includes a drive waveform generating circuit unit and a drive control circuit unit, not shown, and includes an adjusting device that adjusts a drive waveform according to the conditions such as the hardness of a portion to be surgically operated. The drive control unit 110 and a piezoelectric element 51 provided in the interior of the fluid ejecting unit 10 (see Fig. 2) are connected by a connecting cable 130. The infusion solution bag 120 may be accommodated in the interior of he drive control unit 110. In this case, it is preferable to provide a small-sized pump which infuses fluid at a constant pressure in the interior of the drive control unit 110.

Referring now to Fig. 2 to Fig. 4, the fluid ejecting apparatus according to the first embodiment will be described. Fig. 2 is a side cross-sectional view of the fluid ejecting unit according to the first embodiment. In Fig. 2, the fluid ejecting unit 10 includes a fluid chamber 60, an inlet channel 72 that supplies liquid into the fluid chamber 60, and an outlet channel 31 that flows the liquid from the fluid chamber 60 to the opening 30 in the interior of a cylindrical casing 15 formed of an upper frame 70 and a lower frame 80 fixed to each other.

In a state in which the upper frame 70 and the lower frame 80 are secured, a cylindrical fitting portion 12 is formed at a rear end of the casing 15. Then, the inlet channel 72 is brought into communication with the fluid supply tube 20 by the fluid supply tube 20 fitted to the fitting portion 12. Preferably, the outer diameter of the casing 15 substantially matches the outer diameter of the fluid supply tube 20.

The lower frame 80 includes a recess 81 on the surface opposing the upper frame 70. Then, a peripheral edge of a diaphragm 40 is tightly fixed to a peripheral edge of the recess 81. A space defined by the recess 81 and the diaphragm 40 corresponds to the fluid chamber 60.

The upper frame 70 is formed with the inlet channel 72 disposed on the opposite side from the fluid chamber 60 with respect to the diaphragm 40 apart therefrom and extending substantially parallel to the bottom surface of the fluid chamber 60 (recess 81). The fluid chamber 60 and the inlet channel 72 are in communication with each other by a communication channel 65 defined by an upper frame side communication channel 66 and a lower frame side communication channel 77.

The piezoelectric element 51 as an actuator is provided on the surface of the diaphragm 40 on the opposite side from the fluid chamber 60. The upper frame 70 is formed with a recess 75 into a shape avoiding the range in which the piezoelectric element 51 is driven. A check valve 90 is formed at an end portion of the diaphragm 40 on the side of the inlet channel 72. The check valve 90 is provided so as to open and close the upper frame side communication channel 66 which communicates with the inlet channel 72.

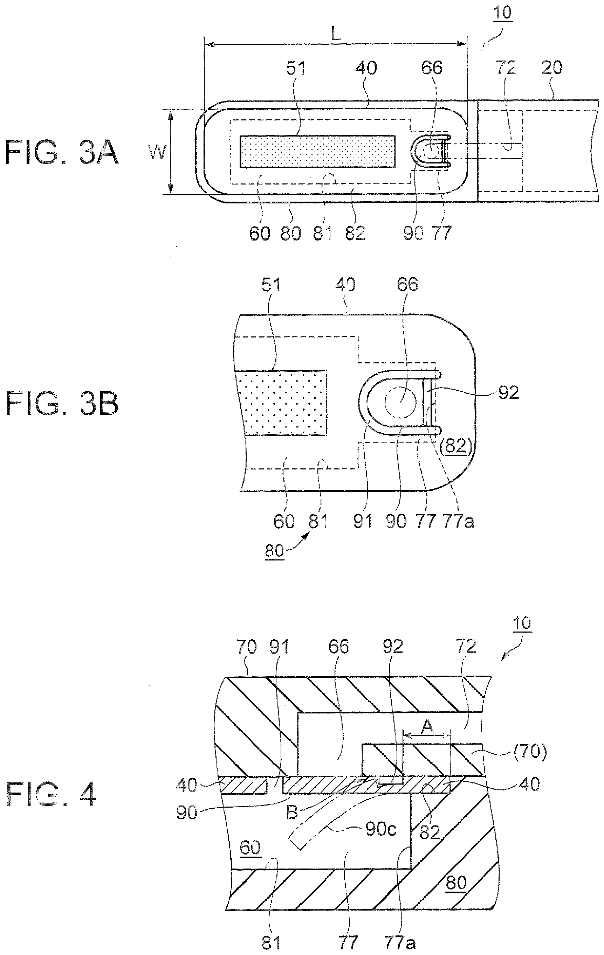

Referring now to Figs. 3 and 4, the shape and the operation of the check valve 90 will be described. Figs. 3Aand 3E show the shape of the check valve according to the first embodiment in plan view. Fig. 3A is a plan view showing the shape of the fluid ejecting unit in plan view in a state of viewing through the upper frame, and Fig. 3B is a partial plan view showing the shape of the check valve in plan view in an enlarged view. Fig. 4 is a partial cross-sectional view showing the operation of the check valve. In Figs. 3A and 3B and Fig. 4, the check valve 90 is formed by providing a slit 91 of a substantially U-shape on the diaphragm 40, and is extended in a peninsula shape from the side of the inlet channel 72 toward the fluid chamber 60. Then, the surface area of the check valve 90 in plan view is larger than the cross-sectional area of the upper frame side communication channel 66.

The lower frame side communication channel 77 having a dimension in the range which does not hinder the operation of the check valve 90 is provided at the end of the recess 81 (fluid chamber 60) on the side of the inlet channel 72 provided on the lower frame 80. More specifically, the size of the lower frame side communication channel 77 is set to a size within a range which does not allow the check valve 90 to come into contact with the recess 81 and with the side wall of the lower frame side communication channel 77 when the upper frame side communication channel 66 is opened.

A thinned portion 92 which is thinner than the diaphragm 40 is provided at a bent portion which is bent when opening and closing the upper frame side communication channel 66 on the surface of the check valve 90 on the side of the upper frame 70 (see Fig. 2). The thinned portion 92 is formed by applying machining process such as half etching to the diaphragm 40 so as to traverse a root portion of the check valve 90.

Subsequently, the fixed state and the operation of the check valve 90 will be described. The peripheral edge of the diaphragm 40 is fixed to a diaphragm fixing surface 82 of the lower frame 80 by means such as adhesion or the like (see Fig. 3A). As shown in Fig. 4, a side wall 77a of the lower frame side communication channel 77 is set to a position apart from the range where the thinned portion 92 is formed. In other words, fixation of the diaphragm 40 (check valve 90) and the diaphragm fixing surface 82 is achieved at a position apart from the range where the thinned portion 92 is formed.

Although the upper frame 70 extends to a range which covers the thinned portion 92, the range of fixation with respect to the check valve 90 is a range apart from the range indicated by A in the drawing where the thinned portion 92 is formed.

The piezoelectric element 51 is provided on the surface of the diaphragm 40 at the substantially center of the fluid chamber 60.

Referring now to Figs. 1 and 2, the flowing action of the liquid in the liquid ejecting apparatus 100 according to the first embodiment will be described. The infusion solution bag 120 is disposed at a position higher than the fluid ejecting unit 10, and liquid is supplied to the fluid chamber 60 via the fluid supply tube 20 at a certain pressure using the pressure difference generated by the difference of the elevation head between the infusion solution bag 120 and the fluid ejecting unit 10. The flow of the supplied liquid is converted into a pulsation flow by the fluid ejecting unit 10, and the liquid passes through the outlet channel 31 and is ejected from the fluid ejection opening 30 in pulses.

Referring now to Figs. 1, 2, and 4, the operation of the fluid ejecting unit 10 will be described. The drive control unit 110 applies a drive waveform formed by the drive waveform generating circuit unit included in the drive control unit 110 to the piezoelectric element 51 from the drive control circuit unit. When supplying the liquid from the inlet channel 72, the check valve 90 is bent to a position 90c shown in Fig. 4, opens the upper frame side communication channel 66, brings the inlet channel 72 and the fluid chamber 60 into communication with each other, and supplies the liquid into the fluid chamber 60.

Assuming that a drive signal is supplied to the piezoelectric element 51, and the piezoelectric element 51 is charged and hence is suddenly contracted, the diaphragm 40 is displaced suddenly into a projecting shape in the direction to reduce the capacity of the fluid chamber 60. Consequently, the pressure in the fluid chamber 60 is increased to a level higher than the pressure supplied from the inlet channel 72, and the check valve 90 closes the upper frame side communication channel 66. In other words, the check valve 90 closes the inlet channel 72. In this manner, the pressure in the fluid chamber 60 is increased further quickly to a several atmospheres when the check valve 90 closes the inlet channel 72. Consequently, a pulsed fluid discharge, that is, high-speed liquid drops 200 are ejected in pulses from the fluid ejection opening 30 through the outlet channel 31.

When the drive signal to be applied to the piezoelectric element 51 is removed, the piezoelectric element 51 is electrically discharged. When the piezoelectric element 51 is electrically discharged, and is restored to the initial state, the diaphragm 40 is suddenly displaced to restore the capacity of the fluid chamber 60 to an initial state. Then, the internal pressure in the fluid chamber 60 is suddenly lowered, or brought into a vacuum state. In this case, since the liquid is supplied from the infusion solution bag 120 to the inlet channel 72 at the constant pressure, the check valve 90 is opened by the pressure difference between the fluid chamber 60 and the inlet channel 72, so that the liquid is supplied to the fluid chamber 60. By repeating the operation of the diaphragm 40 and the check valve 90 as described above, the pulsed liquid drop ejection is continued.

Therefore, according to the first embodiment described above, with the provision of the check valve 90, the liquid is prevented to flow reversely to the inlet channel 72 when the capacity of the fluid chamber 60 is reduced by the diaphragm 40, the internal pressure in the fluid chamber 60 is increased to a required level, and hence the fluid ejecting efficiency is improved.

A plane dimension of the diaphragm 40 in the first embodiment is 1 to 2 mm in width W, several mm in length, and 20 μm in thickness, and the check valve 90 has a further fine shape because it is formed within a plane of the diaphragm 40.

Therefore, according to the first embodiment, the check valve 90 is formed simultaneously with the manufacture of the diaphragm 40, and hence it is not necessary to form the check valve 90 as a single unit, so that manufacture is achieved easily. Since the check valve 90 is attached by attaching the diaphragm 40 to the fluid chamber 60, advantages such as easy-to-handle and improved assembleability are achieved.

By forming the diaphragm 40 and the check valve 90 integrally, the relative positional accuracy between the diaphragm 40 and the check valve 90 is enhanced, thereby achieving an advantage such that the positional accuracy between the upper frame side communication channel 66 and the check valve 90 is improved.

Since the diaphragm 40 is formed of a thin plate, the diaphragm 40 and the check valve 90 are formed easily together in the same process by pressing or etching.

With the provision of the thinned portion 92 at the bent portion of the check valve 90, the check valve 90 is bent with high-sensitivity in response to the pressure difference between the fluid chamber 60 and the inlet channel 72 while having a thickness suitable for the diaphragm 40, closes the inlet channel 72 when reducing the capacity of the fluid chamber 60 and opens the inlet channel 72 when the capacity of the fluid chamber 60 is restored to an initial state.

Since the check valve 90 is bent at the thinned portion 92, the displacement due to bending is not transmitted to the fixing surface (the range A in the drawing or the range of the fixing portion of the diaphragm fixing surface 82). Therefore, when the check valve 90 is bent, an infinitively small gap B (see Fig. 4) is not generated between the fixing portion of the upper frame 70 and the check valve 90.

Accordingly, such events that the fine dust is clogged in the infinitely small gap due to repetition of the bending operation and hence the closing property of the check valve 90 is deteriorated, or a stress is intensively applied to a fulcrum, so that the check valve 90 is destroyed are avoided, and hence the durability of the check valve 90 is improved.

Also, with the provision of the lower frame side communication channel 77 having a size which does not hinder the operation of the check valve 90 at the end of the fluid chamber 60 on the side of the inlet channel 72, the operating range of the check valve 90 is secured in a small range without giving a significant influence on the capacity of the fluid chamber 60, which is advantageous for downsizing.

In addition, the fluid chamber 60 and the inlet channel 72 are disposed apart from each other in the direction of the cross-section as shown in Fig. 2, and the check valve 90 is disposed in the communication channel 65 which communicates the fluid chamber 60 and the inlet channel 72. Therefore, since the check valve 90 can be formed in the same plane as the diaphragm 40, the shape is simplified, and the relative positional accuracy with respect to the opening and closing upper frame side communication channel 66 is improved, so that the inlet channel 72 (the upper frame side communication channel 66) is reliably closed.

### Another Example of First Embodiment

The check valve 90 in the first embodiment preferably assumes the shape as shown in Fig. 5 in order to improve the followability in response to the pressure difference between the fluid chamber 60 and the inlet channel 72.

Fig. 5 is a partial plan view showing the check valve according to another example of the first embodiment. The check valve 90 is formed with narrowed portions 94a and 94b on both side surfaces of the thinned portion 92, and the width is partly narrowed.

In other words, the check valve 90 is improved in followability to open and close the inlet channel in response to the pressure difference between the fluid chamber 60 and the inlet channel 72 by lowering the rigidity of the bent portion by reducing the width of the bent portion.

The narrowed portions 94a and 94b are formed into a smooth arcuate shape. In this arrangement, the intensive stress due to the bending operation of the check valve 90 is avoided, so that the durability is improved.

Structures in which one of the narrowed portions 94a and 94b is provided is also applicable, or in which a through hole is provided at the widthwise center of the bent portion.

### Second Embodiment

Referring now to the drawings, the fluid ejecting apparatus according to a second embodiment will be described. The second embodiment is characterized in that the mode of the communicating portion between the inlet channel and the fluid chamber is different from the first embodiment. Therefore, the points different from the first embodiment will mainly be described. The same functional portions are designated by the same reference numerals.

Fig. 6A is a perspective view showing a part of a lower frame of the fluid ejecting unit according to the second embodiment, and Fig. 6B is a side cross-sectional view showing a part of the fluid ejecting unit according to the second embodiment. In Figs. 6A, and 6B, the lower frame 80 is formed with the recess 81 which constitutes the fluid chamber 60, a connecting hole 68 which is provided along a bottom surface of the recess 81, and the lower frame side communication channel 77 which communicates the inlet channel 72 and the connecting hole 68. The connecting hole 68 and the lower frame side communication channel 77 are in communication with each other via a diaphragm fixing portion 85.

As shown in Fig. 6A, the diaphragm fixing surface 82 is formed in the same plane along the entire peripheral edge of the fluid chamber 60. The cross-sectional shape of the connecting hole 68 is not limited to a square, and may be a circle.

The diaphragm 40 includes the check valve 90, and has the same shape as in the first embodiment (see Figs. 3A and 3B), is secured along the peripheral edge to the diaphragm fixing surface 82, and is secured to the surface of the diaphragm fixing portion 85 (the diaphragm fixing surface 82). In other words, the diaphragm 40 is fixed to the entire circumference of the peripheral edge of the fluid chamber 60, and the root portion of the check valve 90 is secured thereto.

The upper frame side communication channel 66 (that is, the inlet channel 72) is opened and closed by the check valve 90. Then, when the capacity of the fluid chamber 60 is reduced, the upper frame side communication channel 66 is closed by the check valve 90 and, when the capacity of the fluid chamber 60 is restored to the initial state, and the upper frame side communication channel 66 is opened.

In this configuration, since the peripheral edge of the fluid chamber 60 continues completely without the presence of the notch, and the diaphragm 40 can be fixed along the entire circumference of the peripheral edge of the fluid chamber 60, the stable displacement action of the diaphragm 40 is continued.

Although the check valve in the second embodiment has been described with an example of the shape shown in the first embodiment described above (see Figs. 3A and 3B), the invention may be applied to other shapes as well.

### Example 1 in the Second Embodiment

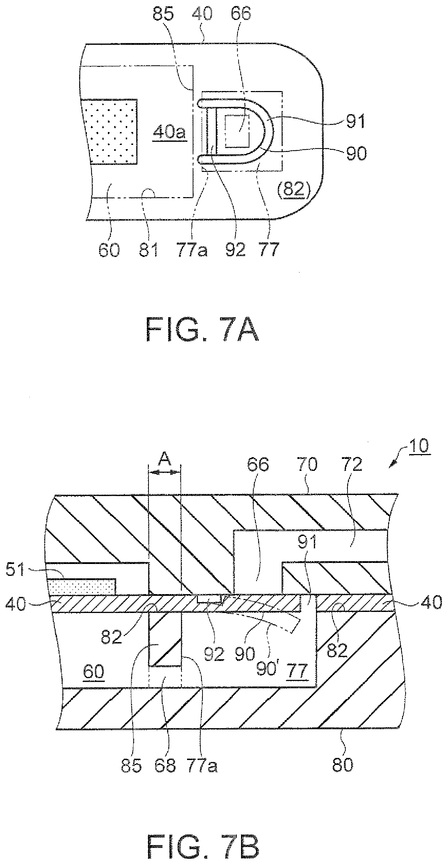

Fig. 7A is a partial plan view showing a diaphragm of the check valve according to Example 1 in the second embodiment, and Fig. 7B is a cross-sectional view showing a part of the fluid ejecting unit of the check valve according to Example 1 in the second embodiment. In Figs. 7A and 7B, the check valve 90 is formed by the slit 91 having a substantially U-shape, has a thinned portion 92 on the side of the fluid chamber 60, and projects toward the upper frame side communication channel 66. The lower frame 80 has a shape similar to that shown in Fig. 6A.

The diaphragm 40 is fixed to the diaphragm fixing surface 82 including the peripheral edge which surrounds the fluid chamber 60. In this case, a side wall 77a on the side of the diaphragm fixing portion 85 of the lower frame side communication channel 77 is set to a position apart from the rage where the thinned portion 92 is formed. In other words, the fixing range of the check valve 90 with respect to the diaphragm fixing surface 82 is located at a position apart from the range where the thinned portion 92 is formed.

Although the upper frame 70 extends to the range which covers the thinned portion 92, the range of fixation of the check valve 90 is a range indicated by A in the drawing, and is a position apart from the range where the thinned portion 92 is formed.

The check valve 90 has a function similar to that in the second embodiment described above, and peripheral edge of the fluid chamber 60 is continued along the entire circumference without the presence of the notch. Accordingly, the diaphragm 40 can be fixed along the entire circumference of the peripheral edge of the fluid chamber 60, and the stable displacement action of the diaphragm 40 is continued.

In the second embodiment, the check valve 90 formed by the substantially U-shaped slit 91 is exemplified. However the shape of the check valve is not limited thereto.

### Example 2 in Second Embodiment

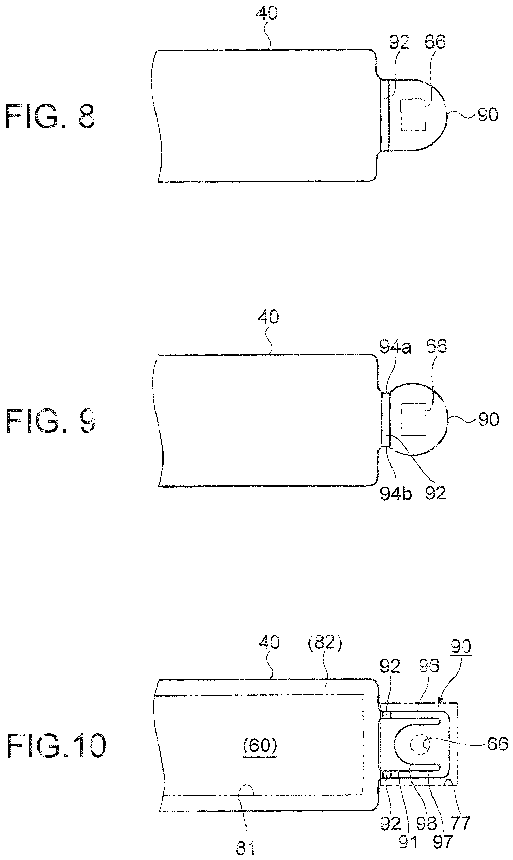

Fig. 8 is a partial plan view showing the check valve according to Example 2 in the second embodiment. The check valve 90 projects from the end of the diaphragm 40 toward the upper frame side communication channel 66 in an peninsula shape. The outline of the check valve 90 is accommodated in the range of the lower frame side communication channel 77 (see Fig. 7A), and has a size which closes the upper frame side communication channel 66.

The thinned portion 92 is provided at the root portion (bent portion) of the check valve 90 at the substantially same portion as Example 1 (see Fig. 7A). The method of fixing the diaphragm 40 is performed on the basis of the same idea as in Example 1 (see Figs. 7A and 7B).

With the shape of the check valve 90 as described above, the same effects and the advantages as in Example 1 in the second embodiment described above are achieved, and the shape is simplified and the manufacture is achieved easily.

### Example 3 in the Second Embodiment

Fig. 9 is a partial plan view showing the check valve according to Example 3 in the second embodiment. The check valve 90 projects from the diaphragm 40 toward the upper frame side communication channel 66 in an peninsula shape. The outline of the check valve 90 is accommodated in the range of the lower frame side communication channel 77 (see Fig. 7A), and has a size which closes the upper frame side communication channel 66.

The check valve 90 is formed with narrowed portions 94a and 94b on both side surfaces of the thinned portion 92, and the width is partly narrowed.

The rigidity of the check valve 90 at the bent portion is reduced by reducing the width of the bent portion. Accordingly, the followability to open and close the inlet channel 72 (the upper frame side communication channel 66) in response to the pressure difference between the fluid chamber 60 and the inlet channel 72 is improved.

The narrowed portions 94a and 94b are preferably formed into a smooth arcuate shape, so that the intensive application of the stress due to the bending operation of the check valve 90 is eliminated and improvement of the durability is achieved.

Structures in which one of the narrowed portions 94a and 94b is provided is also applicable, or in which a through hole is provided at the widthwise center of the bent portion.

### Example 4 in Second Embodiment

Fig. 10 is a partial plan view showing the check valve according to Example 4 in the second embodiment. The check valve 90 includes two supporting arms 96 and 97 projecting from an end of the diaphragm 40 in the direction toward the upper frame side communication channel 66, and a closed portion 98 projecting from distal ends of the supporting arms 96 and 97 toward the end portion of the diaphragm 40.

The sum of the widths of the supporting arms 96 and 97 is smaller than the width of the closed portion 98. Preferably, the width is set to a small value within a range in which the closed portion 98 is positioned in the same plane as the diaphragm 40 in the initial state. Then, the supporting arms 96 and 97 have a width having rigidity and being bendable by the pressure difference between the fluid chamber 60 and the inlet channel 72. The supporting arms 96 and 97 are arranged uniformly on the widthwise both sides of the closed portion 98.

The outline of the check valve 90 is accommodated in the range of the lower frame side communication channel 77 (see Fig. 7A), and has a size which closes the upper frame side communication channel 66.

The fixing structure of the diaphragm 40 including the check valve 90 with respect to the upper frame 70 and the lower frame 80 is achieved by the same relation as the structure shown in Fig. 7B. The supporting arms 96 and 97 each are provided with the thinned portion 92 in the vicinity of the root portion thereof. The positions of the thinned portions 92 correspond to the bent portions of the supporting arms 96 and 97.

Therefore, the check valve 90 is bent at the thinned portion 92 of the supporting arms 96 and 97 by the pressure difference between the fluid chamber 60 and the inlet channel 72 and the closed portion 98 is displaced without deformation to open and close the upper frame side communication channel 66 (the inlet channel 72).

In this configuration, the rigidity of the bent portions may be further reduced, and hence the followability to open and close the upper frame side communication channel 66 in response to the pressure difference between the fluid chamber 60 and the inlet channel 72 is improved.

Since the supporting arms 96 and 97 are disposed uniformly on the widthwise both sides of the closed portion 98, a good bending balance (stress balance) is achieved, and the inclination with respect to the upper frame side communication channel 66 due to the deflection or the like is restrained, so that the upper frame side communication channel 66 is reliably closed.

### Third Embodiment

Referring now to the drawings, the fluid ejecting apparatus according to a third embodiment will be described. The third embodiment is characterized in that the arrangement of the inlet channel and the mode of the check valve are different from the first and second embodiment. Therefore, the points different from the first embodiment will mainly be described. The same functional portions are designated by the same reference numerals.

Fig. 11 is a side cross-sectional view showing a part of the fluid ejecting unit according to the third embodiment. The fluid ejecting unit 10 is provided in such a manner that the inlet channel 72 extends in parallel to the bottom surface of the fluid chamber 60, and is in direct communication with the fluid chamber 60. The check valve 90 is extended by being bent in the substantially vertical direction with respect to the diaphragm fixing surface 82, and opens and closes the inlet channel 72 directly.

The check valve 90 is formed of the slit 91 as in the case of the first embodiment (see Figs. 3A and 3B), and includes the thinned portion 92 on the surface of the bent portion on the side of the lower frame 80. The fixing portion of the check valve 90 between the upper frame 70 and the lower frame 80 is set to a position apart from the thinned portion 92. A plane portion 93 having a surface area which is sufficiently larger than the cross-sectional area of the inlet channel 72 so as to be able to close the inlet channel 72 is provided on the check valve 90 at a distal portion with respect to the thinned portion 92.

As the configuration in the second embodiment described above (see Fig. 6A), the diaphragm 40 can be secured along the entire circumference of the fluid chamber 60 by forming the diaphragm fixing portion 86 having the connecting hole 68 between the inlet channel 72 and the fluid chamber 60 and securing the diaphragm 40 to the diaphragm fixing surface 82 on the upper surface of the diaphragm fixing portion 86.

In this configuration, when the capacity of the fluid chamber 60 is reduced, the check valve 90 closes the inlet channel 72 and, when the capacity of the fluid chamber 60 is restored to the initial state, the check valve 90 is bent to a position 90c to open the inlet channel 72.

The check valve 90 may be formed by forming the thinned portion 92 by machining such as half etching from a deployed state as shown in Fig. 3B (shown by a double-dashed line 90A in Fig. 11) and bending the same to a right angle.

In this configuration, since the inlet channel 72 is in direct communication with the fluid chamber 60, the fluid resistance is reduced in comparison with the structure in which the inlet channel 72 and the fluid chamber 60 are provided apart from each other in the direction of the cross section and are communicated with respect to each other with a communication channel, so that the liquid is allowed to flow easily into the fluid chamber 60, and the dimension in the cross-sectional direction is reduced.

Although the check valve 90 in the third embodiment has been described with an example of the shape formed of the slit 91 as in the first embodiment described above (see Figs. 3A and 3B), the invention may be applied to other shapes as well.

### Example 1 in Third Embodiment

Fig. 12 is a partial plan view showing the check valve according to Example 1 in the third embodiment, and Fig. 13 is a partial cross-sectional view showing a part of the fluid ejecting apparatus. In Figs. 12 and 13, the check valve 90 is projected from the end of the diaphragm 40 toward the inlet channel 72 in a peninsula shape, and is extended by being bent in the substantially vertical direction with respect to the diaphragm fixing surface 82, and opens and closes the inlet channel 72 directly.

The check valve 90 includes the thinned portion 92 on the surface of the bent portion on the side of the upper frame 70. The fixing portion of the check valve 90 between the upper frame 70 and the lower frame 80 is set to a position apart from the thinned portion 92. The plane portion 93 having a surface area which is sufficiently larger than the cross-sectional area of the inlet channel 72 so as to be able to close the inlet channel 72 is provided on the check valve 90 at a distal portion with respect to the thinned portion 92.

The diaphragm fixing portion 86 having the connecting hole 68 is formed between the inlet channel 72 and the fluid chamber 60. The diaphragm 40 is secured to the peripheral edge of the fluid chamber 60 via the diaphragm fixing surface 82 on the upper surface of the diaphragm fixing portion 86.

In this configuration as well, when the capacity of the fluid chamber 60 is reduced, the check valve 90 closes the inlet channel 72 and, when the capacity of the fluid chamber 60 is restored to the initial state, the check valve 90 is bent to a position 90c to open the inlet channel 72.

The check valve 90 may be formed by forming the thinned portion 92 by machining such as half etching from a deployed state as shown in Fig. 11 (shown by a double-dashes line 90A) and bending the same to a right angle.

In this configuration, the same effects and the advantages as in the third embodiment described above are achieved, and the shape of the diaphragm 40 including the check valve 90 is simplified and the manufacture is achieved easily.

### Example 2 in Third Embodiment

Fig. 14 is a partial plan view showing the check valve according to Example 2 in the third embodiment. The deployed shape of the check valve 90 will now be described. The check valve 90 includes the closed portion 98 of the inlet channel extending from the diaphragm 40 so as to project therefrom, and a connecting portion 99 for connecting the closed portion 98 and the end of the diaphragm 40.

The connecting portion 99 extends from the end of the diaphragm 40 and has a width narrower than the closed portion 98. The closed portion 98 extends from the connecting portion 99 to the range which closes the inlet channel 72.

The connecting portion 99 here is bent vertically with respect to the surface of the diaphragm 40. The diaphragm 40 is fixed by the upper frame 70 and the lower frame 80 as shown in Fig. 13 in the state in which the check valve 90 is bent. The connecting portion 99 is formed with the thinned portion 92 on the surface thereof. The position of the thinned portion 92 corresponds to the position of the bent portion of the check valve 90.

Even with the check valve 90 in this configuration, the same effects as in Example 1 in the third embodiment as described above are achieved.

### Fourth Embodiment

Referring now to the drawings, the fluid ejecting apparatus according to a fourth embodiment will be described. The fourth embodiment is characterized by the configuration in which there are two diaphragms and two inlet channels in contrast to the first to third embodiments in which there is one diaphragm provided.

Fig. 15 is a side cross-sectional view showing the fluid ejecting unit according to the fourth embodiment, Fig. 16A is a perspective view of the spacer according to the fourth embodiment, and Fig. 16B is a partial perspective view showing another example of the spacer according to the fourth embodiment. In Fig. 15 and Fig. 16A, the fluid ejecting unit 10 is configured in such a manner that the diaphragm 40 is tightly fixed to a surface 143 of a thin plate-shaped spacer 140, and a diaphragm 41 is tightly fixed to a back surface 144 thereof. The spacer 140 has a frame shape having an opening 141 as shown in Fig. 16A. The opening 141 is sealed on the front and back side with the diaphragm 40 and 41, and hence the fluid chamber 60 defined by the diaphragm 40 as an upper lid, the diaphragm 41 as a lower lid, and the spacer 140 as the side wall is configured.

The outlet channel 31 and the fluid ejection opening 30 are formed at one end of the spacer 140. A communicating portion 142 which continues to the fluid chamber 60 is provided on the side surface opposing the outlet channel 31. Piezoelectric elements 51 and 52 are fixed to the diaphragms 40 and 41 respectively at positions opposing to each other with the intermediary of the fluid chamber 60.

The upper frame 70 is formed with the inlet channel 72 positioned apart from the fluid chamber 60 in the cross-sectional direction and the upper frame side communication channel 66 which communicates the fluid chamber 60 and the inlet channel 72 in the substantially vertical direction. In contrast, the lower frame 80 is formed with the inlet channel 88 positioned apart from the fluid chamber 60 in the cross-sectional direction and a lower frame side communication channel 89 which communicates the fluid chamber 60 and the inlet channel 88 in the substantially vertical direction. The inlet channels 72 and 88 are in communication with the fluid supply tube 20 which is fitted to the rear end of the fluid ejecting unit 10.

Then, the check valves 90 and 95 are provided at openings of the upper frame side communication channel 66 and the lower frame side communication channel 89 on the side of the fluid chamber 60, respectively. The check valve 90 is formed integrally with the diaphragm 40, and the check valve 95 is integrally formed with the diaphragm 41. In this embodiment, the check valve having the same configuration as that in the first embodiment (see Figs. 3A and 3B) descried above may be employed, and the diaphragm 41 may be considered to have a shape which is reversed inside out of the diaphragm 40.

In this configuration, when drive signals having the same phase are applied to the piezoelectric elements 51 and 52, the diaphragms 40 and 41 are displaced with respect to each other to reduce the capacity of the fluid chamber 60. When application of the drive signals to the piezoelectric elements 51 and 52 is stopped, the diaphragms 40 and 41 are both restored to the initial state. When the capacity of the fluid chamber 60 is reduced, the internal pressure of the fluid chamber 60 is increased, so that the check valve 90 closes the inlet channel 72, and the check valve 95 closes the inlet channel 88. When the capacity of the fluid chamber 60 is restored to the initial state, the pressures in the inlet channels 72 and 88 are larger than the internal pressure of the fluid chamber 60, and hence the check valve 90 opens the inlet channel 72 and the check valve 95 opens the inlet channel 88, so that the liquid is supplied to the fluid chamber 60.

In this configuration, the ratio of capacity reduction of the fluid chamber 60 is enhanced by providing the diaphragms 40 and 41 respectively on the surfaces opposing to each other for one fluid chamber 60, and driving the same in the same phase. Since there are two inlet channels which communicate with the fluid chamber 60, a sufficient amount of liquid supply is achieved.

Since the check valves 90 and 95 are provided at the respective inlet channels, a reverse flow of the liquid to the inlet channels 72 and 88 is prevented and the pressure in the fluid chamber 60 is enhanced, so that a stronger liquid drop ejection is effectively achieved.

Although a configuration in which the two inlet channels 72 and 88 are provided for one fluid chamber has been described as an example in this embodiment, a configuration in which one of the inlet channels 72 and 88 is provided so as to communicate with the fluid chamber 60 is also applicable. In this case, it is also possible to prepare two pairs of the inlet channel and the check valve, maintain one of the check valves in the closed state, and releases the fixation of the closed check valve as needed to achieve the two-pair configuration.

In this embodiment, a technical idea in the second embodiment described above (see Figs. 6A and 6B) may be applied. For example, as shown in Fig. 16B, a communication hole 146 which penetrates from the surface 143 to the back surface 144 is formed at a position apart from the opening 141 of the spacer 140 in the direction of the plane, and a connecting hole 145 which communicates from the opening 141 to the communication hole 146 is provided. The communication hole 146 communicates with the upper frame side communication channel 66 and the lower frame side communication channel 89 shown in Fig. 15.

In this configuration, since a diaphragm fixing surface 143a is formed on the front surface side of the spacer 140, and a diaphragm fixing surface 143b is formed on the back surface side thereof, so that the diaphragms 40 and 41 are respectively fixed over the entire circumference of the peripheral edge of the opening 141, and the stable displacement action of the diaphragms 40 and 41 are continued.

In the structure employing the spacer 140 as in the fourth embodiment, the fluid ejecting unit having a configuration having only a single inlet channel is easily achieved by opening only one of the front surface side or the back surface side of the communication hole 146.

### Fifth Embodiment

Referring now to the drawings, the fluid ejecting apparatus according to a fifth embodiment will be described. The fifth embodiment is characterized in that the configuration of the inlet channel is different from the configuration in the first to fourth embodiments described above. Although the fifth embodiment may be applied to the configuration of the fluid ejecting unit described in the first to fourth embodiments described above respectively, an example of being applied to the first embodiment (see Fig. 2) will be described.

Fig. 17A is a side cross-sectional view of the fluid ejecting unit according to the fifth embodiment, and Fig. 17B is a cross-sectional view taken along the line C-C in Fig. 17A. In Figs. 17A and 17B, the inlet channel 72 is configured with a groove 78 which is formed along the periphery of the upper frame 70 along the direction of length thereof and an inner periphery 21 of the fluid supply tube 20.

The groove 78 has a cross-sectional area required as the inlet channel. The opening of the groove 78 is sealed by fitting the fluid supply tube 20 to a fitting portion 13 of the casing 15, so that the inlet channel 72 is formed.

Even with the configuration in which the two inlet channels are provided as in the fourth embodiment described above (see Fig. 15), the two inlet channels are configured by forming two grooves and fitting the fluid supply tube 20.

In this configuration, since the inlet channel 72 is formed by the groove 78 provided on the periphery of the fluid ejecting unit 10 (upper frame 70), the groove 78 may be formed by cutting process, which is easier than forming into a thin tube shape.

There are also advantages such that the flexibility of the size of the cross-sectional area of the inlet channel 72 is enhanced, and the adjustment of the amount of liquid supply is facilitated.

### Sixth Embodiment

Referring now to the drawings, a sixth embodiment will be described. Although the configuration of the check valve has been mainly described in the first to the third embodiments described above, a configuration of the diaphragm 40 will be described in the sixth embodiment.

Figs. 18 and 19 show a configuration of the diaphragm according to the sixth embodiment. Fig. 18 is a plan view and Fig. 19 is a cross-sectional view taken along the line A-A in Fig. 18. The shape of the check valve 90 in the first embodiment is used as an example here, and description is omitted.

In Figs. 18 and 19, the diaphragm 40 is formed with a waveform structure 42 in the periphery of the piezoelectric element 51. The waveform structure 42 can be formed by embossing process on the surface of the diaphragm 40.

The waveform structure 42 is formed at an intermediate position between the piezoelectric element 51 and the side wall of the recess 81 which constitutes the fluid chamber 60. Although the waveform structure 42 is exemplified as a single ridge configuration in Figs. 18 and 19, it may be configured as multiple ridges.

In the case of the diaphragm 40 having a flat structure shown in the first to third embodiments described above, even though the rigidity is low in the initial deformation caused by the piezoelectric element 51, an inplane tensile stress is generated in the diaphragm 40 as the outplane deformation increases.

The inplane stress increases the rigidity of the diaphragm 40. Therefore, the inplane stress is restrained with the provision of the waveform structure 42, so that efficient conversion of the displacement of the piezoelectric element 51 due to the expansion or contraction thereof into reduction of the capacity of the fluid chamber 60 is enabled even though the plane surface area of the diaphragm 40 is small.

The waveform structure 42 may also be applied to the diaphragms shown in the fourth and fifth embodiments.

The liquid ejecting apparatus 100 according to the illustrative embodiments and embodiments of the invention may be employed in various manner in drawing using ink or the like, cleaning of the precise substances or structures, incision or excision of the substances, or surgical knifes, and specifically suitable as a surgical operation instrument used by installing at the distal end of the catheter used by inserting in the blood vessel and removing the thrombus and the like, or as a surgical operation instrument for incising or excising anatomy.

## Claims

1. A fluid ejecting apparatus comprising:
a fluid ejecting unit (10) configured to eject fluid from a fluid ejecting opening (30) in pulses by reducing the capacity of a fluid chamber (60) by at least a first diaphragm (40);
an inlet channel (72) that supplies the fluid to the fluid chamber (60); and
a check valve (90) disposed between the inlet channel (72) and the fluid chamber (60),
wherein the check valve (90) closes the inlet channel (72) when the capacity of the fluid chamber (60) is reduced, and the check valve (90) is bent open when the capacity of the fluid chamber (60) is expanded from the reduced state to open the inlet channel (72),
**characterized in that** the fluid chamber (60) is defined by a frame-shaped spacer (140) having an opening (141) penetrating in the direction of thickness,
**in that** the first diaphragm (40) and a second diaphragm (41) are disposed on both surfaces of the spacer (140) for sealing the opening,
and **in that** the check valve (90) is formed on at least one of the first and second diaphragms.

2. The fluid ejecting apparatus according to Claim 1, wherein the check valve is formed with a slit (91).

3. The fluid ejecting apparatus according to Claim 1 or 2, wherein the check valve (90) includes a thinned portion (92) at a bent portion in the direction of thickness thereof.

4. The fluid ejecting apparatus according to Claim 3, wherein the check valve (90) is fixed at a position apart from a range where the thinned portion (92) is formed.

5. The fluid ejecting apparatus according to any one of the preceding Claims, wherein a communicating portion (66) having a size in a range which does not hinder the operation of the check valve (90) is provided at an end of the fluid chamber (60) on the side of the inlet channel (72).

6. The fluid ejecting apparatus according to any one of the preceding Claims, wherein the inlet channel (72) is disposed on the opposite side from the fluid chamber (60) with respect to said at least one of the first and second diaphragms (40, 41) so as to apart therefrom, and the check valve (90) is disposed in a communication channel (65) which communicates the fluid chamber and the inlet channel.

7. The fluid ejecting apparatus according to Claim 6, wherein the communication channel (65) is provided at a position apart from a peripheral edge of the fluid chamber (60), and is in communication with the fluid chamber (60) via a connecting hole (68).

8. The fluid ejecting apparatus according to Claim 6, wherein the check valve (90) projects to a position covering from an end portion of said at least one of the first and second diaphragms (40, 41) to the communicating portion.

9. The fluid ejecting apparatus according to Claim 3, wherein the check valve (90) has a width at the bent portion smaller than portions other than the bent portion.

10. The fluid ejecting apparatus according to Claim 6, wherein the check valve (90) includes two supporting arms projecting from an end of said at least one of the first and second diaphragms (40, 41) in the direction toward the communication channel (65), and a closed portion positioned between the two supporting arms and projecting from distal ends of the two supporting arms toward the end of said at least one of the first and second diaphragms (40, 41), and the sum of the widths of the supporting arms is smaller than the width of the closed portion, and the closed portion opens and closes the communication channel (72) by the supporting arms being bent.

11. The fluid ejecting apparatus according to any one of the preceding Claims, wherein the inlet channel (72) is provided substantially in parallel with a bottom surface (81) of the fluid chamber, and is brought into communication directly with the fluid chamber (60), and the check valve (90) is extended by being bent in the substantially vertical direction with respect to the fixing surface (82) of said frame on which the peripheral edge of the at least one of the first and second diaphragms is fixed , and opens and closes the inlet channel (72).

12. The fluid ejecting apparatus according to any one of the preceding Claims, wherein two inlet channels communicating with the fluid chamber are provided, and wherein two check valves are provided on the first and second diaphragms and open and close the two inlet channels respectively.

13. The fluid ejecting apparatus according to any one of the preceding Claims, wherein the inlet channel (72) is defined by a groove (78) provided along the outer periphery of the fluid ejecting unit (10) and an inner periphery (21) of a fluid supply tube (20) fitted so as to cover the groove.

14. The fluid ejecting apparatus according to any one of the preceding Claims, wherein at least one of the first and second diaphragms is provided with a waveform structure (42) having projections and depressions in the direction of thickness of said at least one of the first and second diaphragms around a piezoelectric element for displacing said at least one of the first and second diaphragms.

15. A surgical operating instrument comprising the fluid ejecting apparatus according to any one of the preceding Claims.

## Patentansprüche

1. Fluidausgabevorrichtung, aufweisend:
eine Fluidausgabeeinheit (10), die eingerichtet ist, ein Fluid von einer Fluidausgabeöffnung (30) in Pulsen auszugeben, indem die Kapazität einer Fluidkammer (60) durch zumindest eine erste Membran (40) reduziert wird,
einen Einlasskanal (72), der das Fluid zur Fluidkammer (60) zuführt, und
ein Rückschlagventil (90), das zwischen dem Einlasskanal (72) und der Fluidkammer (60) angeordnet ist,
wobei das Rückschlagventil (90) den Einlasskanal (72) schließt, wenn die Kapazität der Fluidkammer (60) reduziert wird, und das Rückschlagventil (90) offen gebogen wird, wenn die Kapazität der Fluidkammer (60) vom reduzierten Zustand expandiert wird, um den Einlasskanal (72) zu öffnen,
**dadurch gekennzeichnet, dass**
die Fluidkammer (60) durch einen rahmenförmigen Abstandshalter (140) definiert ist, der eine Öffnung (141) aufweist, die in die Dickenrichtung dringt,
dadurch, dass die erste Membran (40) und eine zweite Membran (41) an beiden Flächen des Abstandshalters (140) zum Abdichten der Öffnung angeordnet sind,
und dadurch, dass das Rückschlagventil (90) an zumindest einer von der ersten und zweiten Membran ausgebildet ist.

2. Fluidausgabevorrichtung gemäß Anspruch 1, bei der das Rückschlagventil mit einem Spalt (91) ausgebildet ist.

3. Fluidausgabevorrichtung gemäß Anspruch 1 oder 2, bei der das Rückschlagventil (90) einen dünneren Abschnitt (92) an einem Biegeabschnitt in der Dickenrichtung hiervon aufweist.

4. Fluidausgabevorrichtung gemäß Anspruch 3, bei der das Rückschlagventil (90) an einer Position fixiert ist, die von einem Bereich beabstandet ist, wo der dünnere Abschnitt (92) ausgebildet ist.

5. Fluidausgabevorrichtung gemäß einem der vorangegangenen Ansprüche, bei der ein Kommunikationsabschnitt (66) mit einer Größe in einem Bereich, die den Betrieb des Rückschlagventils (90) nicht behindert, an einem Ende der Fluidkammer (60) an der Seite des Einlasskanals (72) vorgesehen ist.

6. Fluidausgabevorrichtung gemäß einem der vorangegangenen Ansprüche, bei der der Einlasskanal (72) an der gegenüberliegenden Seite von der Fluidkammer (60) bezüglich der zumindest einen der ersten und zweiten Membran (40, 41) angeordnet ist, um hiervon beabstandet zu sein, und das Rückschlagventil (90) in einem Verbindungskanal (65) angeordnet ist, der die Fluidkammer und den Einlasskanal verbindet.

7. Fluidausgabevorrichtung gemäß Anspruch 6, bei der der Verbindungskanal (65) an einer von einer Umfangskante der Fluidkammer (60) beabstandeten Position vorgesehen ist, und mit der Fluidkammer (60) via einer Verbindungsausnehmung (68) in Verbindung ist.

8. Fluidausgabevorrichtung gemäß Anspruch 6, bei der das Rückschlagventil (90) zu einer Position vorsteht, die von einem Endabschnitt der zumindest einen der ersten und zweiten Membran (40, 41) zum Verbindungsabschnitt reicht.

9. Fluidausgabevorrichtung gemäß Anspruch 3, bei der das Rückschlagventil (90) eine Breite am Biegeabschnitt aufweist, die kleiner ist als Abschnitte abgesehen vom Biegeabschnitt.

10. Fluidausgabevorrichtung gemäß Anspruch 6, bei der das Rückschlagventil (90) zwei Trägerarme, die von einem Ende der zumindest einen der ersten und zweiten Membran (40, 41) in der Richtung zum Verbindungskanal (65) vorstehen, und einen geschlossener Abschnitt aufweist, der zwischen den zwei Trägerarmen positioniert ist und von distalen Enden der zwei Trägerarme zum Ende der zumindest einen der ersten und zweiten Membran (40, 41) vorsteht, und die Summe der Breiten der Trägerarme kleiner als die Breite des geschlossenen Abschnitts ist, und der geschlossene Abschnitt den Verbindungskanal (72) öffnet und schließt, indem die Trägerarme gebogen werden.

11. Fluidausgabevorrichtung gemäß einem der vorangegangen Ansprüche, bei der der Einlasskanal (72) im Wesentlichen parallel mit einer Bodenfläche (81) der Fluidkammer vorgesehen ist, und direkt mit der Fluidkammer (60) in Verbindung gebracht wird, und das Rückschlagventil (90) verlängert wird, indem es in der im Wesentlichen vertikalen Richtung bezüglich der Fixierfläche (82) des Rahmens gebogen wird, an dem die Umfangskante der zumindest einen der ersten und zweiten Membran fixiert ist, und den Einlasskanal (72) öffnet und schließt.

12. Fluidausgabevorrichtung gemäß einem der vorangegangenen Ansprüche, bei dem zwei Einlasskanäle, die mit der Fluidkammer kommunizieren, vorgesehen sind, und wobei zwei Rückschlagventile an der ersten und zweiten Membran vorgesehen sind, und die zwei Einlasskanäle entsprechend öffnen und schließen.

13. Fluidausgabevorrichtung gemäß einem der vorangegangenen Ansprüche, bei der der Einlasskanal (72) durch eine Nut (78) definiert ist, die entlang des Außenumfangs der Fluidausgabeeinheit (10) vorgesehen ist, und ein Innenumfang (21) eines Fluidzuführrohrs (20), das so angebracht ist, um die Nut abzudecken.

14. Fluidausgabevorrichtung gemäß einem der vorangegangenen Ansprüche, bei der zumindest eine der ersten und zweiten Membran mit einer Wellenformstruktur (42) versehen ist, die Vorsprünge und Vertiefungen in der Dickenrichtung der zumindest einen der ersten und zweiten Membran um ein piezoelektrisches Argument zum Versetzen der zumindest einen der ersten und zweiten Membran aufweist.

15. Chirurgisches Betätigungselement, umfassend die Fluidausgabevorrichtung gemäß einem der vorangegangenen Ansprüche.

## Revendications

1. Appareil d'éjection de fluide comprenant :
une unité d'éjection de fluide (10) configurée pour éjecter du liquide à partir d'une ouverture d'éjection de fluide (30) par impulsions en réduisant la capacité d'une chambre de fluide (60) par au moins une première membrane (40) ;
un canal d'entrée (72) qui fournit le fluide à la chambre de fluide (60) ; et
un clapet de non-retour (90) disposé entre le canal d'entrée (72) et la chambre de fluide (60),
dans lequel le clapet de non-retour (90) ferme le canal d'entrée (72) quand la capacité de la chambre liquide (60) est réduite, et le clapet de non-retour (90) est plié ouvert lorsque la capacité de la chambre de fluide (60) est étendue à partir de l'état réduit pour ouvrir le canal d'entrée (72),
**caractérisé en ce que** la chambre de fluide (60) est définie par une entretoise en forme de cadre (140) ayant une ouverture (141) pénétrant dans le sens de l'épaisseur,
**en ce que** la première membrane (40) et une seconde membrane (41) sont disposées sur les deux surfaces de l'entretoise (140) pour sceller l'ouverture,
et **en ce que** le clapet de non-retour (90) est formé sur au moins une des première et seconde membranes.

2. Appareil d'éjection de fluide selon la revendication 1, dans lequel le clapet de non-retour est formé avec une fente (91).

3. Appareil d'éjection de fluide selon la revendication 1 ou 2, dans lequel le clapet de non-retour (90) inclut une partie amincie (92) au niveau d'une partie pliée dans le sens de l'épaisseur de ce dernier.

4. Appareil d'éjection de fluide selon la revendication 3, dans lequel le clapet de non-retour (90) est fixé au niveau d'une position espacée d'une plage où la partie amincie (92) est formée.

5. Appareil d'éjection de fluide selon l'une quelconque des revendications précédentes, dans lequel une partie de communication (66) ayant une taille dans une plage qui ne gêne pas le fonctionnement du clapet de non-retour (90) est prévue au niveau d'une extrémité de la chambre de fluide (60) sur le côté du canal d'entrée (72).

6. Appareil d'éjection de fluide selon l'une quelconque des revendications précédentes, dans lequel le canal d'entrée (72) est disposé sur le côté opposé de la chambre de fluide (60) par rapport à ladite au moins une des première et seconde membranes (40, 41) de façon à s'en écarter, et le clapet de non-retour (90) est disposé dans un canal de communication (65) qui fait communiquer la chambre de fluide et le canal d'entrée.

7. Appareil d'éjection de fluide selon la revendication 6, dans lequel le canal de communication (65) est prévu au niveau d'une position espacée d'un bord périphérique de la chambre de fluide (60), et est en communication avec la chambre de fluide (60) via un trou de connexion (68).

8. Appareil d'éjection de fluide selon la revendication 6, dans lequel le clapet de non-retour (90) dépasse jusqu'à une position couvrant d'une partie d'extrémité de ladite au moins une des première et seconde membranes (40, 41) jusqu'à la partie de communication.

9. Appareil d'éjection de fluide selon la revendication 3, dans lequel le clapet de non-retour (90) a une largeur au niveau de la partie pliée plus petite que des parties autres que la partie pliée.

10. Appareil d'éjection de fluide selon la revendication 6, dans lequel le clapet de non-retour (90) inclut deux bras de support dépassant d'une extrémité de ladite au moins une des première et seconde membranes (40, 41) dans le sens vers le canal de communication (65), et une partie fermée positionnée entre les deux bras de support et dépassant des extrémités distales des deux bras de support vers l'extrémité de ladite au moins une des première et seconde membranes (40, 41), et la somme des largeurs des bras de support est plus petite que la largeur de la partie fermée, et la partie fermée ouvre et ferme le canal de communication (72) par les bras de support étant pliés.

11. Appareil d'éjection de fluide selon l'une quelconque des revendications précédentes, dans lequel le canal d'entrée (72) est prévu sensiblement en parallèle avec une surface de fond (81) de la chambre de fluide, et est amené en communication directement avec la chambre de fluide (60), et le clapet de non-retour (90) est étendu en étant plié dans la direction sensiblement verticale par rapport à la surface de fixation (82) dudit cadre sur lequel le bord périphérique de l'au moins une des première et seconde membranes est fixé, et ouvre et ferme le canal d'entrée (72).

12. Appareil d'éjection de fluide selon l'une quelconque des revendications précédentes, dans lequel deux canaux d'entrée communiquant avec la chambre de fluide sont prévus, et dans lequel deux clapets de non-retour sont prévus sur les première et seconde membranes et ouvrent et ferment respectivement les deux canaux d'entrée.

13. Appareil d'éjection de fluide selon l'une quelconque des revendications précédentes, dans lequel le canal d'entrée (72) est défini par une rainure (78) prévue le long de la périphérie extérieure de l'unité d'éjection de fluide (10) et une périphérie intérieure (21) d'un tube d'alimentation en fluide (20) ajusté de façon à couvrir la rainure.

14. Appareil d'éjection de fluide selon l'une quelconque des revendications précédentes, dans lequel au moins une des première et seconde membranes est munie d'une structure de forme d'onde (42) ayant des parties en saillie et des dépressions dans le sens de l'épaisseur de ladite au moins une des première et seconde membranes autour d'un élément piézoélectrique pour déplacer ladite au moins une des première et seconde membranes.

15. Instrument chirurgical opérationnel comprenant l'appareil d'éjection de fluide selon l'une quelconque des revendications précédentes.
